# EUROPEAN PATENT SPECIFICATION

(11) **EP 3 748 362 B1**
(45) Date of publication and mention of the grant of the patent: **11.10.2023**
(21) Application number: 19382470.3
(22) Date of filing: 06.06.2019
(51) Int. Cl.: G01N 33/68, G01N 33/82

(54) **IN VITRO METHOD FOR DETECTING RENAL DISEASE**
IN-VITRO-VERFAHREN ZUR DETEKTION EINER NIERENERKRANKUNG
PROCÉDÉ IN VITRO POUR LA DÉTECTION D'UNE MALADIE RÉNALE

(43) Date of publication of application: 09.12.2020
(73) Proprietor: Fundación Instituto de Investigación Sanitaria Fundación Jiménez Díaz, 28040 Madrid (ES); Universidad Autónoma de Madrid, 28049 Madrid (ES)
(72) Inventor: ORTIZ ARDUAN, Alberto, 28040 Madrid (ES); GONZÁLEZ-PARRA, Emilio, 28040 Madrid (ES); MORGADO PASCUAL, José Luis, 28040 Madrid (ES); EGIDO DE LOS RÍOS, Jesús, 28040 Madrid (ES); RUIZ-ORTEGA, Marta, 28040 Madrid (ES); RAYEGO-MATEOS, Sandra, 28040 Madrid (ES)
(74) Representative: Hoffmann Eitle

(56) References cited:
- WO-A1-2006/019659
- WO-A1-2013/041913
- WO-A1-2014/113291
- BISHOP GAIL A ET AL: "TRAF3 as a Multifaceted Regulator of B Lymphocyte Survival and Activation.", FRONTIERS IN IMMUNOLOGY 2018, vol. 9, 2018, page 2161, XP055615465, ISSN: 1664-3224

## Description

### FIELD OF THE INVENTION

The present invention refers to the medical field. Particularly, the present invention refers to the *in vitro* use of TRAF3 protein levels, determined in peripheral blood mononuclear cells (PBMCs) isolated from blood samples obtained from healthy donors and patients, for detecting renal disease, for predicting the response of patients suffering from renal disease to a treatment with vitamin D analogues or derivatives, or for deciding or recommending whether to treat patients suffering from renal disease with vitamin D analogues or derivatives.

### STATE OF THE ART

Chronic kidney disease (CKD) is a type of kidney disease in which there is gradual loss of kidney function over a period of months or years. At an early stage, there are typically no symptoms. However, in a later stage, leg swelling, feeling tired, vomiting, loss of appetite, or confusion may develop. Complications may include heart disease, high blood pressure, bone disease or anemia.

Causes of CKD include diabetes, hypertension, glomerulonephritis, and polycystic kidney disease. Risk factors include a family history of the condition. Diagnosis is generally by blood tests to measure the glomerular filtration rate and urine tests to measure albumin. Further tests such as an ultrasound or kidney biopsy may be done to determine the underlying cause.

Screening at-risk people is recommended. Initial treatments may include medications to manage blood pressure, sugar levels in blood, and lower cholesterol. NSAIDs should be avoided. Other recommended measures include staying active and certain dietary changes. Severe disease may require replace therapies such as hemodialysis, peritoneal dialysis, or a kidney transplant. Treatments for anemia and bone disease may also be required.

CKD affected 753 million people globally in 2016, including 417 million females and 336 million males. In 2015 it resulted in 1.2 million deaths, up from 409,000 in 1990. The causes that contribute to the greatest number of deaths are high blood pressure at 550,000, followed by diabetes at 418,000, and glomerulonephritis at 238,000.

Diagnosis of CKD is largely based on history, examination and urine dipstick: glomerular filtration and/or albuminuria normalized with respect to levels or concentration of urinary creatinine. It is important to differentiate CKD from acute kidney injury (AKI) because AKI can be reversible. One diagnostic clue that helps differentiate CKD from AKI is a gradual rise in serum creatinine (over several months or years) as opposed to a sudden increase in the serum creatinine (several days to weeks). In many CKD patients, previous kidney disease or other underlying diseases are already known. A significant number of patients present CKD of unknown cause. Patients may progress CKD to end-stage renal damage (ESRD).

Apart from controlling other risk factors, the goal of therapy is to slow down or halt the progression of CKD. Control of blood pressure and treatment of the original disease are the broad principles of management.

Nowadays, the definition of reliable biomarkers of renal disease progression and the effective treatment thereof is an unmet clinical need.

The reference *[*TRAF3 delays cyst formation induced by NF-κB signaling. Sun L, Hu C, Zhang X. IUBMB Life. 2017 Mar;69(3):170-178. doi: 10.1002/iub.1601. Epub 2017 Feb 10*]* has been identified, but the approach disclosed therein clearly differs from the present invention. Sun L., et al 2017 only provides *in vitro* experiments in a transformed cell line wherein TRAF3 protein is overexpressed to determine its association with cell proliferation and apoptosis, both characteristics of renal polycystosis. Please note that renal disease, particularly CKD, assayed in the present invention can be considered as a different medical indication as compared with renal polycystosis. It should be noted that CKD is characterized by inflammation and fibrosis, whereas apoptosis in human CKD has not been reported. In fact, renal polycytosis is a very specific hereditary kidney disease that has a dominant autosomal genetic etiology. This occurs due to mutations in the PKD1 or PKD2 genes, which code for the polycystin-1 and polycystin-2 (PC-1 and PC-2) proteins. On the other hand, please note that Sun L., et al 2017 does not provide reliable and reproducible conclusions. It is important to consider that usual controls such as isolated transfection reagents or empty vectors have not been used in Sun L., et al 2017, so the results provided in this publication regarding the overexpression of TRAF3 could be non-conclusive. It is also important to note that, while in the present invention TRAF3 protein levels are measured in patients with CKD, renal levels of TRAF3 in injured kidneys are not determined in Sun L., et al 2017. Last but not least, please note that in the present invention minimally invasive samples are used because TRAF3 levels are determined in blood cells obtained from the patients with CKD. Consequently, the method described in the present invention is a non-invasive procedure, which allows monitoring the patients by measuring TRAF3 levels over the time. However, Sun L., et al 2017 is completely silent about the possibility of using blood samples.

Thus, the present invention is focused on providing a solution to a clear unmet medical need, which is the finding of reliable biomarkers of renal disease progression and the effective treatment thereof. The present invention solves this problem by using TRAF3 protein levels, determined in peripheral blood mononuclear cells (PBMCs) isolated from blood samples obtained from the patient, for detecting renal disease, for predicting the response of patients suffering from renal disease to a treatment with vitamin D analogues or derivatives, or for deciding or recommending whether to treat patients suffering from renal disease with vitamin D analogues or derivatives.

### DESCRIPTION OF THE INVENTION

### Brief description of the invention

The present invention clearly shows (see **Examples 1** and **2)** that TRAF3 protein levels are significantly reduced in patients suffering from renal disease in different scenarios: 1) In peripheral blood mononuclear cell (PMBCs) from patients with ESRD versus healthy subjects, 2) in kidney tissue from animal models in which renal damage was induced by administration of TWEAK, folic acid (FA) or unilateral ureteral obstruction (UUO) (3 different and accepted models of experimental CKD) and 3) *in vitro* studies developed in tubular epithelial cells (representative renal cells of human [HK2] or murine origin [MCT]) and peripheral blood mononuclear cell (PMBCs) in which renal damage was induced by the administration of TWEAK. Moreover, the present invention also demonstrates that the use of vitamin D analogues or derivatives, for example paricalcitol, gives rise to an increase of TRAF3 protein levels which brings about a positive therapeutic response in renal damage induced by TWEAK or other type of models of acute kidney injury (AKI) or chronic kidney disease (CKD). In this case, a reduction of the expression of pro-inflammatory markers associated with renal damage was observed including a novel therapeutic modulation of NF-kB2 signaling pathway.

Particularly, the first embodiment described herein refers to an *in vitro* method for determining the level of TRAF3 protein in a patient which comprises: a) Isolating peripheral blood mononuclear cells (PBMCs) from blood samples obtained from the patient, and b) determining the level of TRAF3 by using reagents able to detect TRAF3. The second embodiment of the present invention refers to an *in vitro* method for detecting renal disease which comprises: a) Determining at least TRAF3 protein level following the method of the first embodiment and b) wherein if the level determined in step (a) is statistically lower than the level determined in healthy control subjects, this is an indication that the patient is suffering from renal disease.

The third embodiment of the present invention refers to an *in vitro* method for predicting the response of patients suffering from renal disease to a treatment with vitamin D analogues or derivatives which comprises: a) Determining at least TRAF3 protein level following the first embodiment after the administration of the vitamin D analogue or derivative, and b) wherein if the level determined in step (a) is statistically higher than the expression level determined before the administration of the vitamin D analogue or derivative, this is an indication that the patient responds to the treatment.

The fourth embodiment of the present invention refers to an *in vitro* method for deciding or recommending whether to treat patients suffering from renal disease with vitamin D analogues or derivatives which comprises: a) Determining at least TRAF3 protein level following the first embodiment and b) wherein if the level determined in step (a) is statistically lower than the level determined in healthy control subjects, a treatment with vitamin D analogues or derivatives is recommended.

The sixth embodiment of the present invention refers to the *in vitro* use of the TRAF3 protein level determined in peripheral blood mononuclear cells (PBMCs) isolated from blood samples obtained from the patient for detecting renal disease, for predicting the response of patients suffering from renal disease to a treatment with vitamin D analogues or derivatives, or for deciding or recommending whether to treat patients suffering from renal disease with vitamin D analogues or derivatives.

The seventh embodiment not part of the present invention refers to a kit suitable for measuring the level of TRAF3 in a patient which comprises: a) Tools for isolating peripheral blood mononuclear cells (PBMCs) from blood samples obtained from the patient; and b) reagents able to detect TRAF3.

The eight embodiment not part of the of the present invention refers to the use of the above defined kit for detecting renal disease, for predicting the response of patients suffering from renal disease to a treatment with vitamin D analogues or derivatives, or for deciding or recommending whether to treat patients suffering from renal disease with vitamin D analogues or derivatives.

In a preferred embodiment, the renal disease is clinically manifested as acute kidney injury (AKI) or chronic kidney disease (CKD), or the renal disease has progressed to end stage renal disease (ESRD). For the interpretation of the above cited terms AKI, CKD and ESRD please refer to the following scientific publication: *[*The Spanish Society of Nephrology (SENEFRO) commentary to the Spain GBD 2016 report: Keeping chronic kidney disease out of sight of health authorities will only magnify the problem. Ortiz A, Sanchez-Niño MD, Crespo-Barrio M, De-Sequera-Ortiz P, Fernández-Giráldez E, García-Maset R, Macía-Heras M, Pérez-Fontán M, Rodriguez-Portillo M, Salgueira-Lazo M, Sánchez-Álvarez E, Santamaría-Olmo R, Simal Blanco F,Pino-Pino MD. Nefrologia. 2019 Jan-Feb;39(1):29-34.doi: 10.1016/j.nefro.2018.09.002. Epub 2018 Nov 28*].*

For the purpose of the present invention, the following terms are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare TRAF3 levels obtained according to the method of the invention with a defined threshold value. Furthermore, retrospective measurement of TRAF3 levels (or scores) in properly banked historical subject samples may be used in establishing these threshold values. The term "reference control level", according to the present invention, may refer to two different situations:
   ∘ "TRAF3 level determined in patients suffering from renal disease": In this regard, the patient would have been successfully treated with vitamin D analogues or derivatives if the level of TRAF3 after the administration of the treatment is statistically higher as compared with the level determined in control patients suffering from renal disease.
   ∘ "TRAF3 level determined in control healthy subjects": In this regard, the patients would be identified as patients suffering from renal disease, and consequently selected for receiving a treatment with a vitamin D analogue or derivative, if their level of TRAF3 is statistically lower as compared with the level determined in control healthy subjects.
- By "therapeutically effective dose or amount" of a composition comprising vitamin D analogues or derivatives is intended an amount that, when administered as described herein, brings about a positive therapeutic response in a subject having renal disease. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

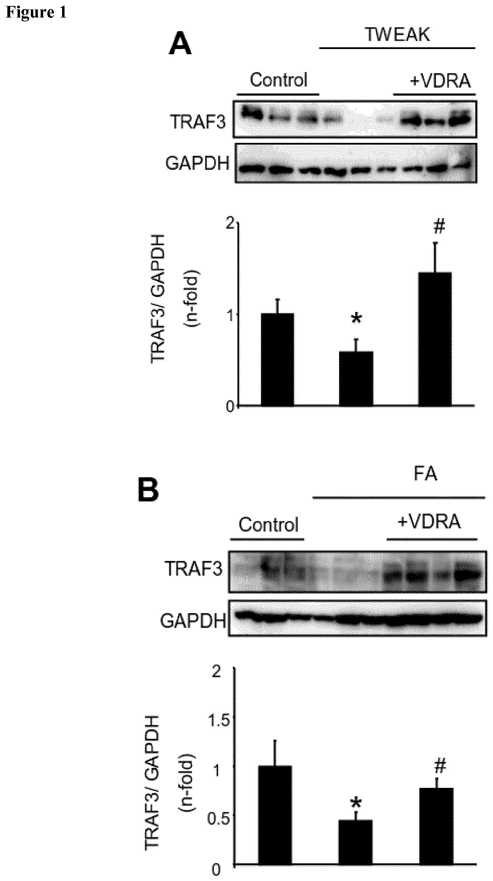

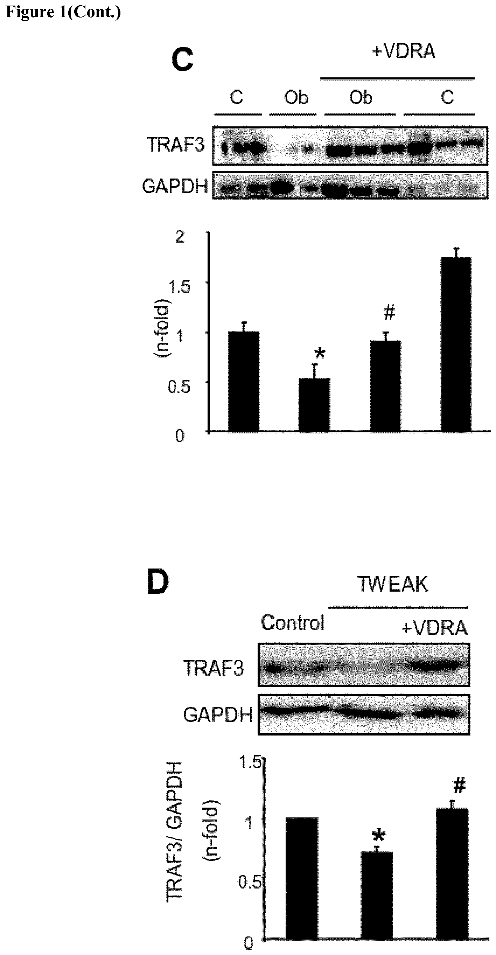

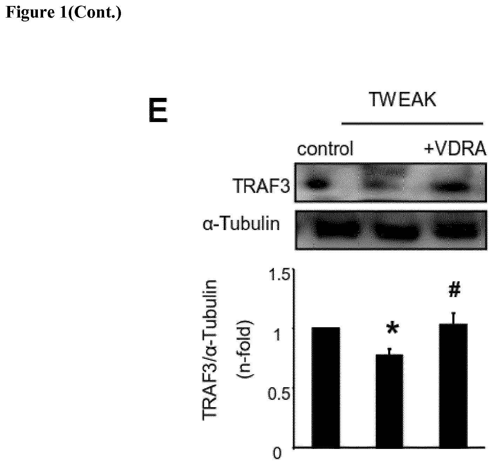

**Figure 1****. Paricalcitol restored TRAF3 in several models of renal damage.** Evaluation of TRAF3 levels in experimental renal damage, assessed by Western blot. **A.** Mice were treated with paricalcitol 750 ng/kg/day starting 48 hours before TWEAK 0.5 µg administration and sacrificed 24 hours later. **B.** Mice were treated with paricalcitol 25 ug/Kg/day, starting 24 hours before folic acid (FA) 300 mg/kg or vehicle (sodium bicarbonate 0.3 mol/L), and studied after 24 hours. **C.** Mice were treated with paricalcitol 750 ng/Kg/day, starting 48 hours before unilateral ureteral obstruction (UUO) and studied after 5 days. **D.** Paricalcitol restored TRAF3 levels in cytokine-stimulated cells. Human tubular cells (HK2) **(D)** or PBMCs from healthy donors **(E)** were pretreated with paricalcitol (15 umol/L) 48 hours before TWEAK stimulation (100 ng/ml) for 24 hours. TRAF3 protein levels by western blot. Data expressed as mean±SEM of 6-8 animals per group or of 3 in vitro experiments. *p<0.05 vs control; #p<0.05 vs ESRD cells or injured-kidney or TWEAK-treated cells.

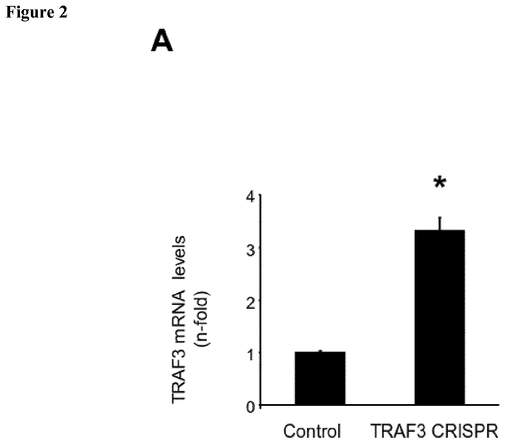

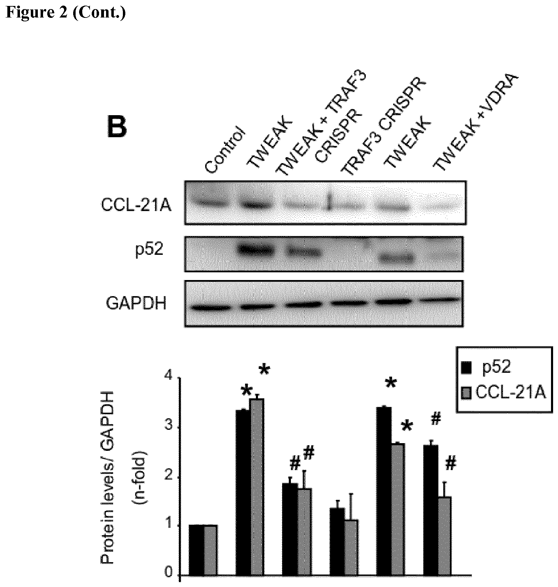

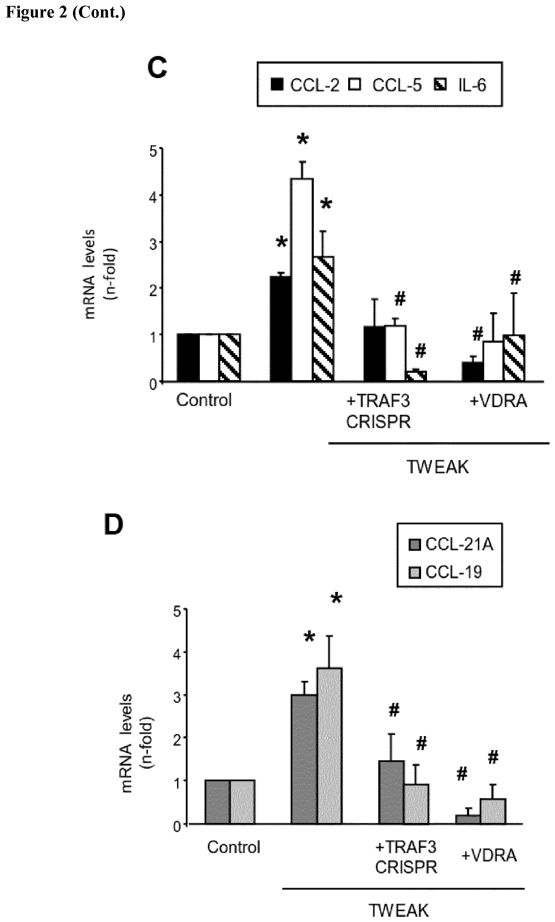

**Figure 2****. TRAF3 overexpression mimics paricalcitol actions.** TRAF3 overexpression was achieved in cultured cells using an activator TRAF3/CRISPR/Cas9 DNA plasmid. HK2 Cells were stimulated with recombinant human soluble TWEAK 100 ng/ml. In some experiments cells were preincubated for 48 hours with paricalcitol 15 µmol/L prior to TWEAK stimulation. **A.** TRAF3 mRNA levels are increased in cells transfected with CRISPR/Cas9 TRAF3 activation plasmid as evaluated by real time PCR. **B.** NF-κB2 pathway activation was assessed by western blot of NFκB2 subunit p52 and the NFxB2-regulated cytokine CCL21A. **C** and **D.** Gene expression in MCTs of the proinflammatory factors CCL2, CCL5 and IL-6 (C) or CCL21A and CCL19 **(D)** were evaluated by real time PCR. Data expressed as mean±SEM of 3-5 independent experiments. *p<0.05 vs control; #p<0.05 vs TWEAK-treated cells.

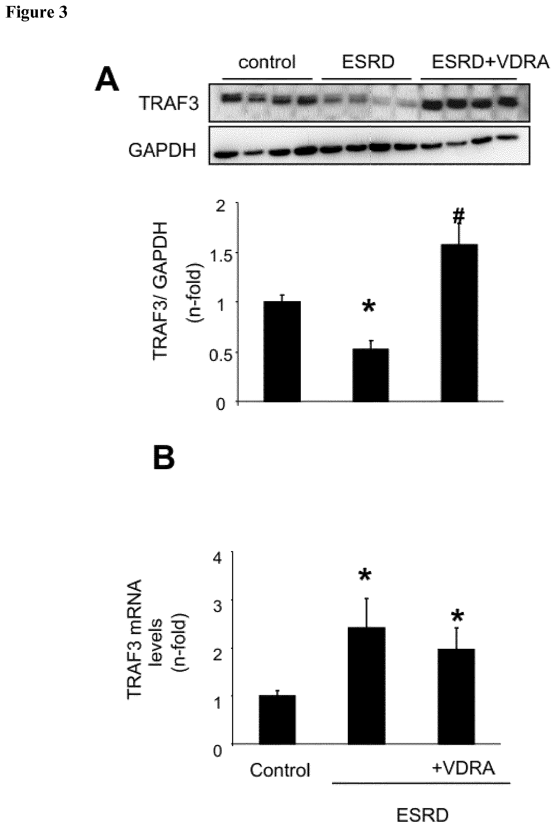

**Figure 3****. Paricalcitol restored TRAF3 levels in ESRD patients and in cultured peripheral blood mononuclear cells (PBMCs). A.** TRAF3 protein levels in PBMC from ESRD patients treated or not with paricalcitol (15 umol/L) were determined by western blot. Number of patients 5-8 per group. *p<0.05 vs control; #p<0.05 vs ESRD cells. **B.** TRAF3 mRNA levels in PBMC from ESRD patients treated or not with paricalcitol were determined by real time RT-PCR. Number of patients 5-8 per group. *p<0.05 vs control. Number of patients 5 per group. *p<0.05 vs control.

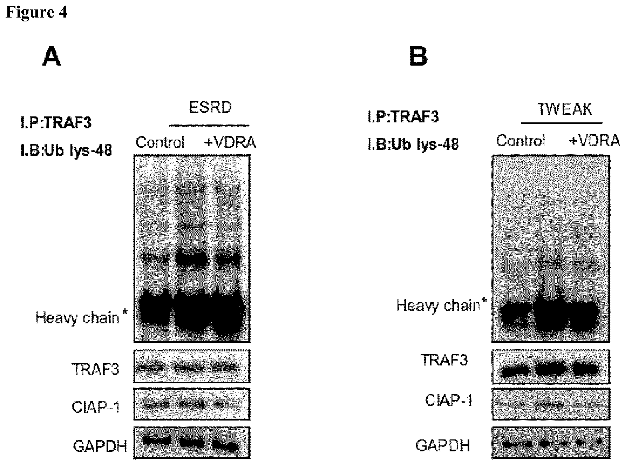

**Figure 4****. Modulation of TRAF3 ubiquitination and CIAP1 complexing was involved in paricalcitol restoration of TRAF3 levels. A.** In PBMCs from ESRD in hemodialysis, treated or not with vitamin D receptor activator (VDRA) paricalcitol, TRAF3 was immunoprecipitated (IP) by an anti-TRAF3 antibody followed by SDS-PAGE and western blotting (IB) against anti-Ubiquitin-Lys-48, TRAF3 and CIAP1 was performed to analyze the possible linkage between them. Representative experiment. TRAF3 antibody was used as loading control. Number of patients 5-8 per group. **B.** TWEAK-induced TRAF3 ubiquitination was prevented by paricalcitol in HK2 cells stimulated with TWEAK (100 ng/ml) and treated or not with paricalcitol (15 umol/L). TRAF3 was immunoprecipitated (IP) by an anti-TRAF3 antibody followed by SDS-PAGE and western blotting (IB) against anti-Ubiquitin-Lys-48, TRAF3 and CIAP1 was performed to analyze the possible linkage between them. Representative experiment. TRAF3 antibody was used as loading control. Figures show a representative IP experiment out of 3 performed.

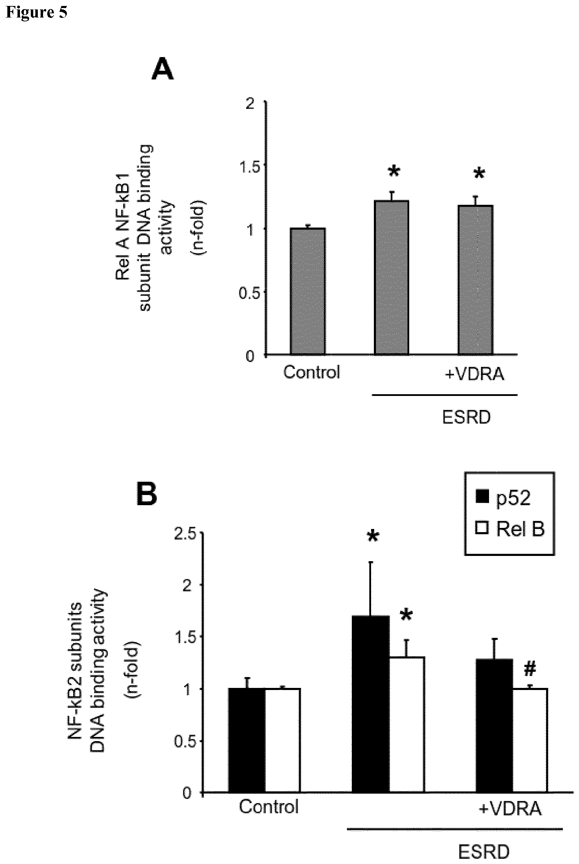

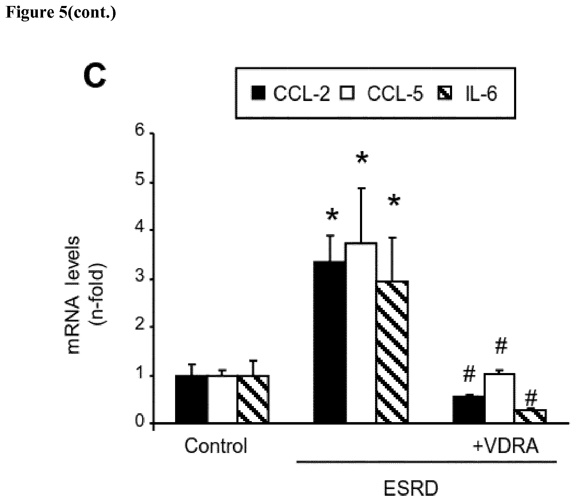

**Figure 5****. Paricalcitol (VDRA) inhibits non-canonical NF-κB signaling pathway (NF-xB2), but not the canonical NF-κB signaling pathway (NF-κB1) in PBMCs from end-stage renal disease patients (ESRD) on hemodialysis.** Activation of canonical and non-canonical NF-κB pathways in PBMCs was assessed by p65 **(A)** or p52 and RelB **(B)** DNA binding activities, respectively. Number of patients: 5-8 per group. *p<0.05 vs healthy control; #p<0.05 vs non-paricalcitol ESRD patients. **(C)** Paricalcitol decreased proinflammatory genes in PBMCs from end-stage renal disease (ESRD) patients in hemodialysis. PBMC from control individuals or ESRD patients on and off paricalcitol were isolated and tested for proinflammatory gene expression. Gene expression was assessed by real time PCR. Number of patients: 5-8 per group.

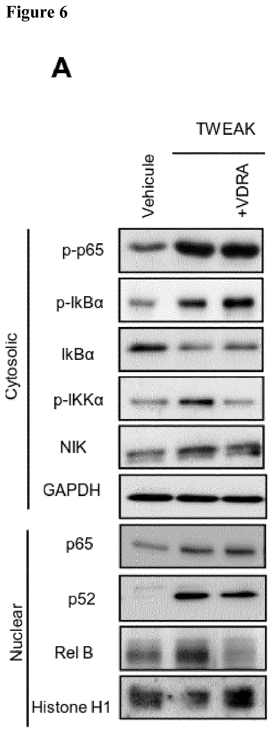

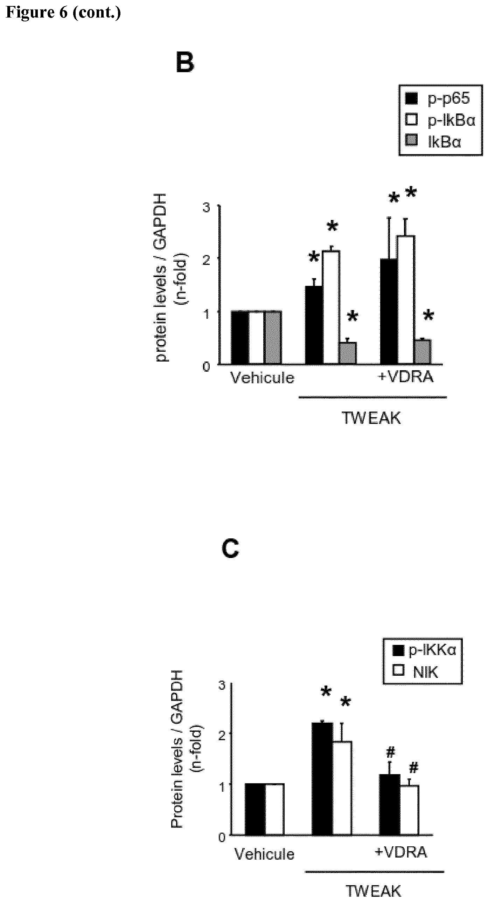

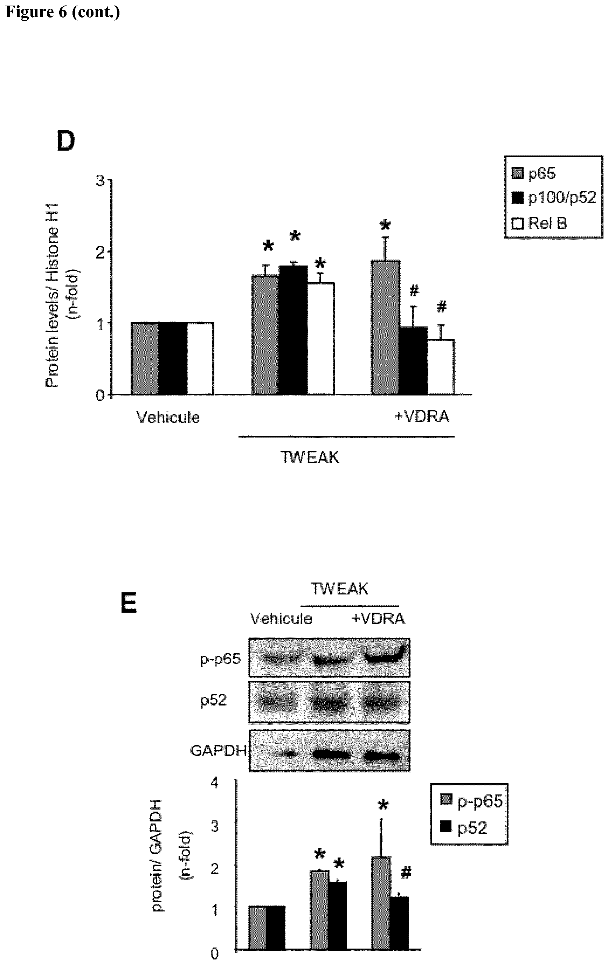

**Figure 6****. Paricalcitol decreased non-canonical NF-xB2 activation but not canonical NF-κB activation induced by TWEAK in renal tubular epithelial cell and in PBMC from healthy donors. A-D.** Human tubular cells were pretreated with 12 µM paricalcitol for 48 hours prior to stimulation with 100 ng/ml TWEAK for 15 min (NF-κB1 pathway) or 6 hours (NF-κB2 pathway). Activation of the canonical NF-κB 1 was assessed by IκBα or p65 phosphorylation in total protein extracts, and p65 levels in cytosolic or nuclear fractions **(A** and **B).** Activation of the non-canonical NF-κB2 pathway was assessed by evaluating NIK and phosphorylated IKK-α levels in total protein extracts **(A** and **C)** and the subcellular location of p100/p52 and Rel B subunits **(A** and **D).** GAPDH and Histone H1 were used as total and nuclear protein loading controls, respectively. **A.** Representative Western blot. **B, C** and **D.** Quantification of protein levels expressed as mean±SEM of 4 experiments. *p<0.05 vs control. # p<0.05 vs TWEAK. **E.** Human PBMCs cells were pretreated with 12 µM paricalcitol during 24 hours, before stimulation with 100 ng/ml TWEAK (E). Activation of canonical NF-κB 1 activation was assessed as phosphorylated p65 in cytosolic fractions and non-canonical activation NF-κB2 by p52 levels. *p<0.05 vs control; #p<0.05 vs TWEAK -stimulated.

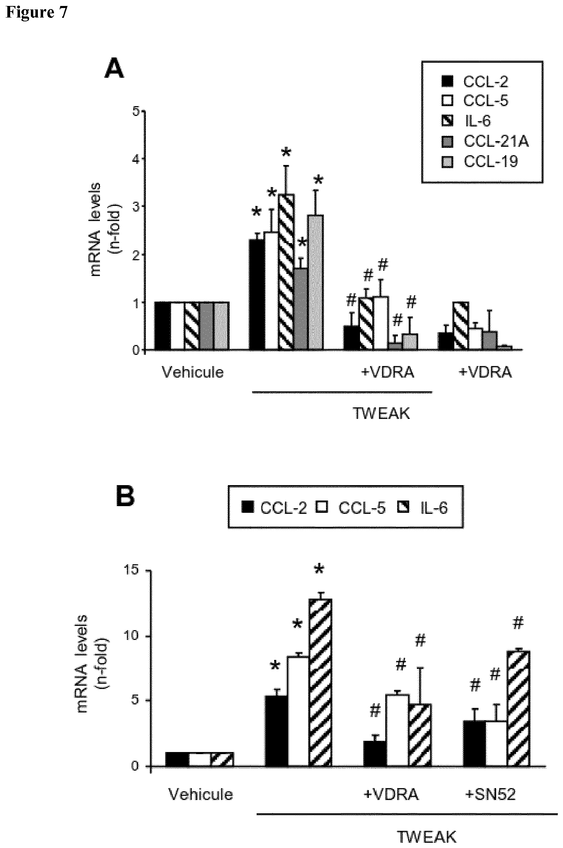

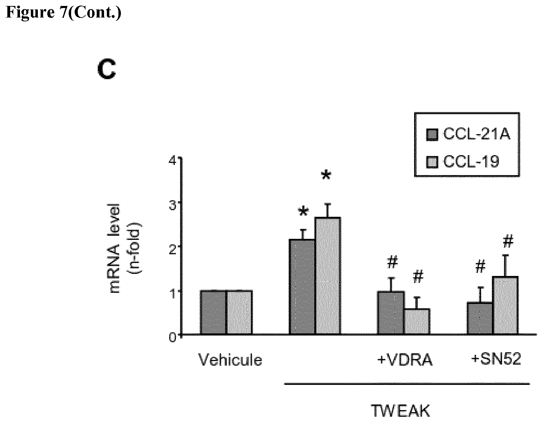

**Figure 7****. Paricalcitol decreased proinflammatory genes, cytokine-stimulated PBMC from control individuals or tubular cells in cultured. A.** PBMCs from control individuals on and off paricalcitol were isolated and tested for proinflammatory gene expression. **B.** PBMC from control individuals were isolated and stimulated in culture with cytokines. **C.** Murine cultured tubular epithelial cells. Cultured PBMCs or murine tubular epithelial cells were stimulated with 100 ng/mL TWEAK for 6 hours. Cells were pre-incubated for 48 hours with 15 µmol/L paricalcitol or 60 ng/ml SN52. Gene expression was assessed by real time PCR. Number. Data expressed as mean±SEM of 3-6 experiments. *p<0.05 vs control; #p<0.05 vs TWEAK-treated cells.

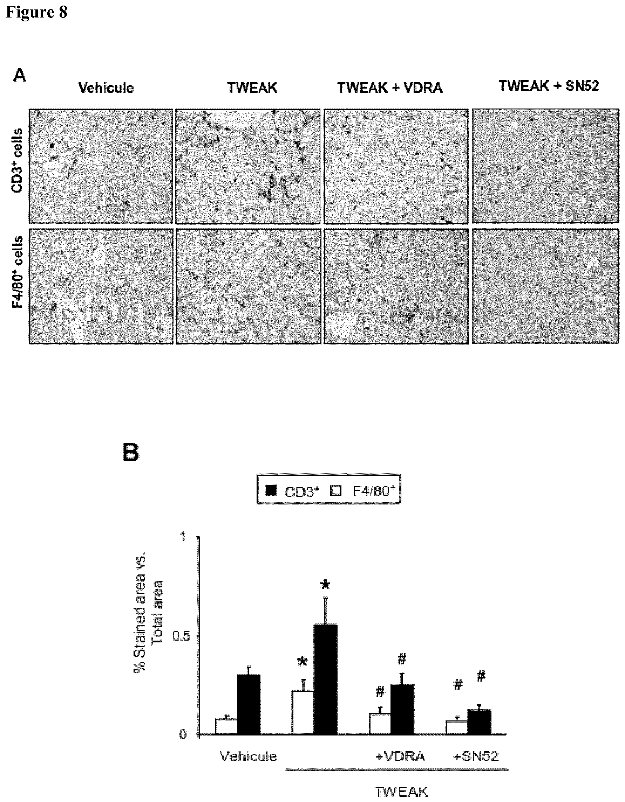

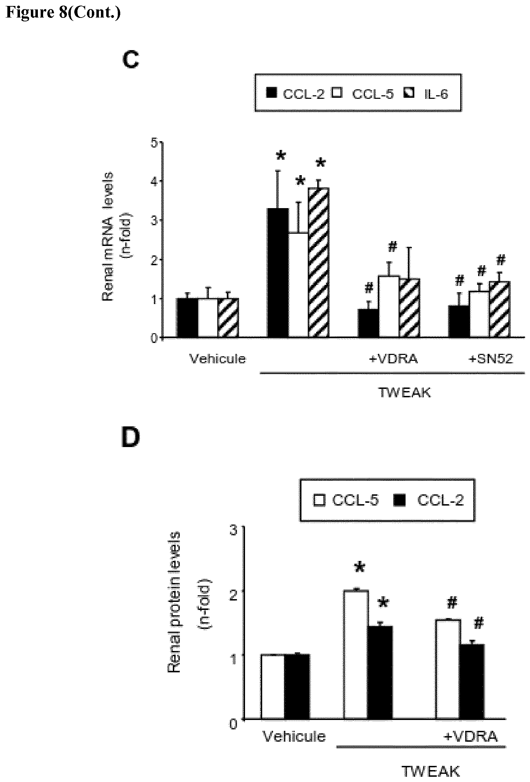

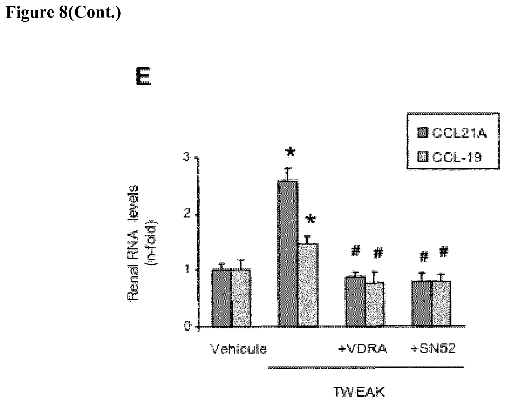

**Figure 8****. Paricalcitol or NF-xB2 blockade ameliorated TWEAK-induced renal inflammation.** C57BL/6 mice were treated with paricalcitol 750 ng/Kg/day or the NF-κB2 inhibitor SN52 0.7 mg/day (two doses; day -1; day 0) starting 48 hours before TWEAK 0.5 µg, and sacrificed 24 hours after TWEAK administration. **A.** Immunohistochemistry using anti-F4/80 and anti-CD3 identified monocyte/macrophages and T lymphocytes, respectively. Representative animal from each group. Magnification 200X. **B.** Staining quantification **C.** RNA was obtained from total renal extracts and proinflammatory gene expression levels (CCL2, CCL5 and IL6) were determined by Real Time PCR. **D.** Renal CCL2 and CCL5 protein levels were evaluated by ELISA. **E.** NF-xB2-regulated cytokines, CCL21A and CCL19 gene expression levels were determined by Real Time PCR. Data expressed as mean±SEM of 8-10 animals per group. *p<0.05 vs control. # p<0.05 vs TWEAK.

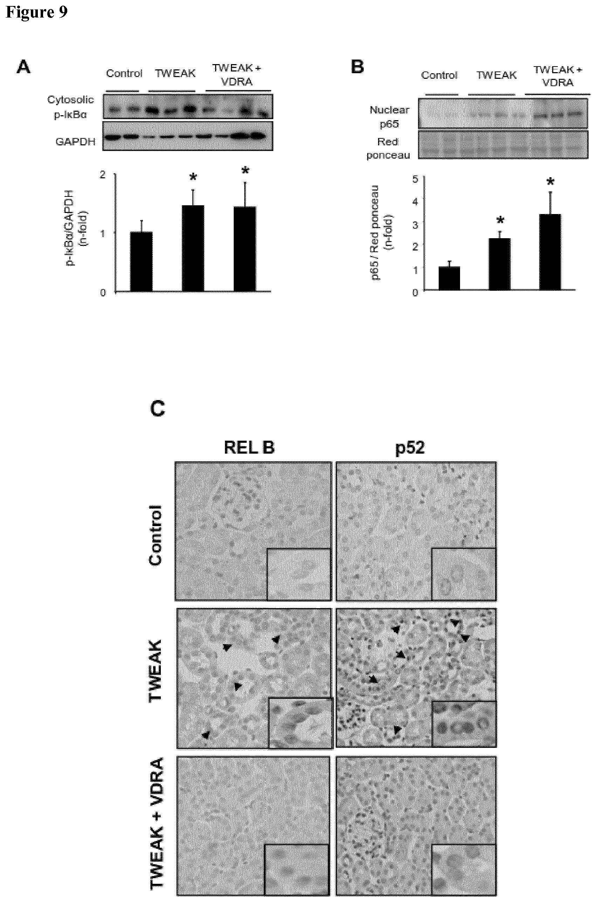

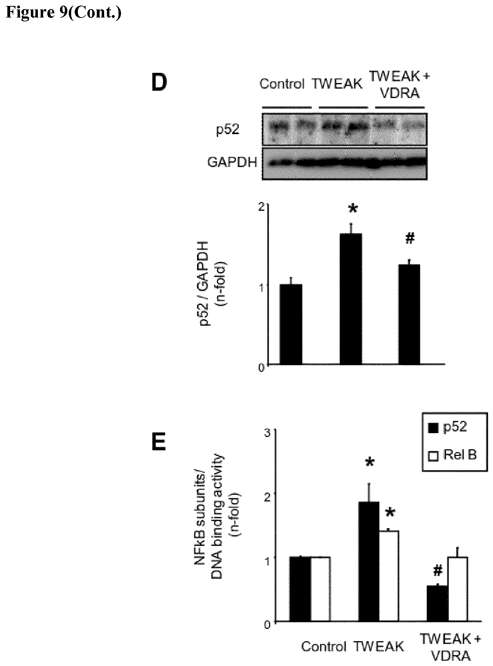

**Figure 9****. Paricalcitol only inhibits TWEAK-induced non-canonical NF-κB activation *in vivo.*** Mice were treated with paricalcitol 750 ng/Kg/day starting 48 hours before TWEAK 0.5 µg, and sacrificed 24 hours after TWEAK administration. **A** and **B.** In TWEAK-injected mice, increased cytosolic phosphorylated IκBα levels **(A)** and nuclear p65 levels **(B)** were found, but were not modulated by paricalcitol treatment, suggesting that paricalcitol did not modulate NF-κB 1 activation. GAPDH and Ponceau Red were used as loading controls. Data expressed as mean ± SEM of 8-10 mice per group. *p<0.05 vs control. **C.** Nuclear location of p52 and Rel B in renal tubules of TWEAK-injected mice assessed by immunohistochemistry. **D.** Western blot assessment of NF-κB2 activation (p52 levels) in whole kidney protein extracts. **E.** In renal nuclear protein extracts, p52 and RelB NF-κB2 DNA binding activity were measured by ELISA. All data expressed as mean±SEM of 8-10animals per group. *p<0.05 vs control. # p<0.05 vs TWEAK.

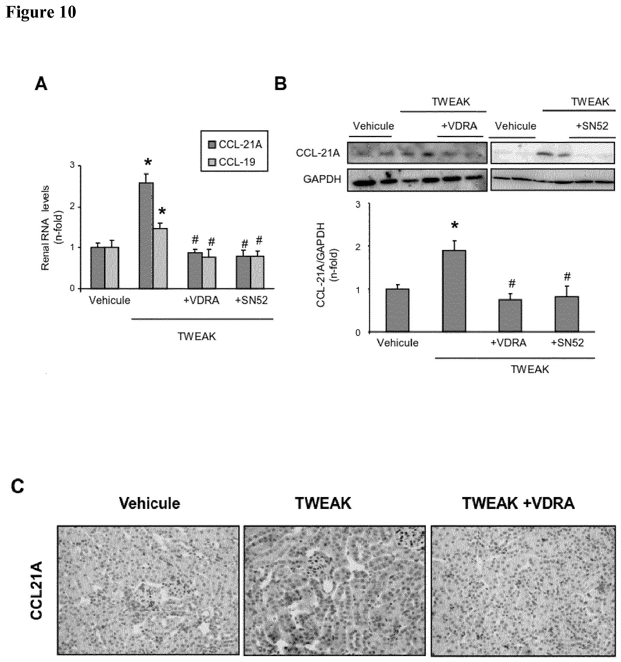

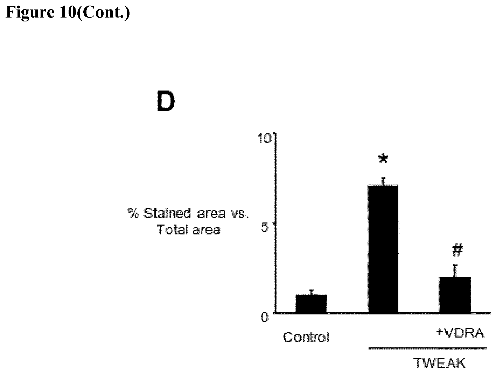

**Figure 10****. Paricalcitol inhibits TWEAK-induced upregulation of specific NF-xB2 targets in the kidney.** Mice were treated with paricalcitol 750 ng/Kg/day or the NF-κB2 inhibitor SN52 0.7 mg/ day (two doses; day -1; day 0) starting 48 hours before TWEAK 0.5 µg, and sacrificed 24 hours after TWEAK administration. **A.** RNA was obtained from total renal extracts and gene expression of the NF-κB2-regulated cytokines CCL21A and CCL19 was determined by Real Time PCR. **B.** CCL21A protein levels were evaluated by western blot in total kidney proteins. GAPDH was used as loading control. **C.** CCL21A expression in the kidney. Representative mouse from each group. **D.** Quantification of CCL21A stained area vs total area. Data expressed as mean ± SEM of 8-10 mice per group. *p<0.05 vs control. # p<0.05 vs TWEAK.

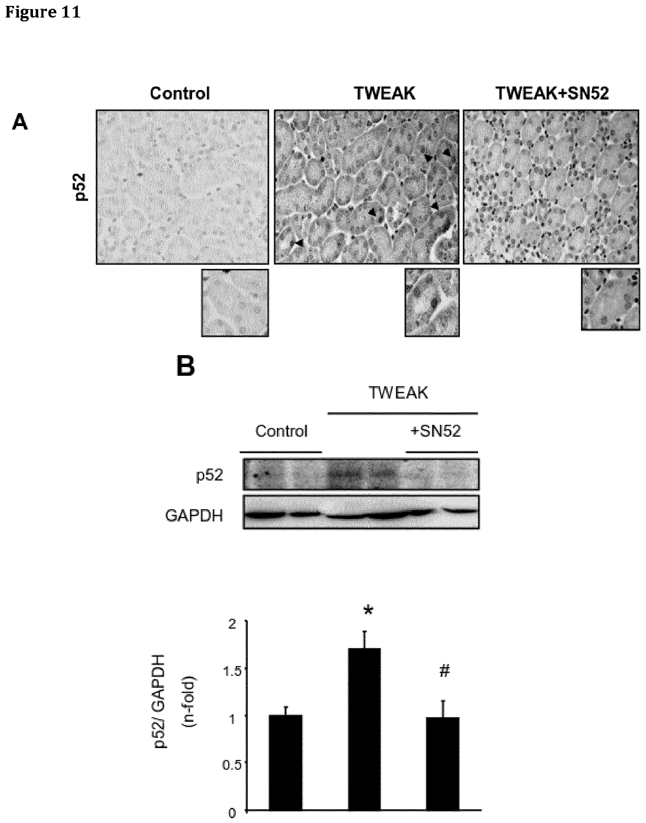

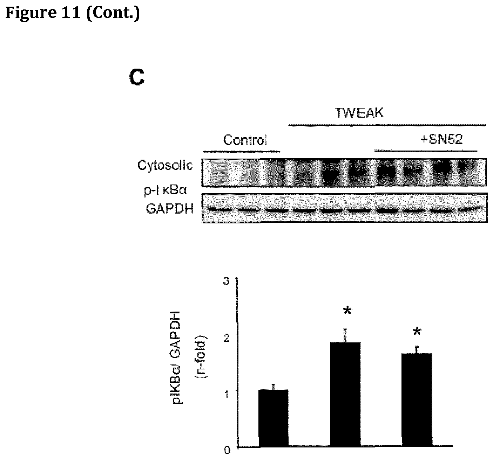

**Figure 11****. SN52 peptide blocks NF-κB2 activation and p52 nuclear translocation in a model of TWEAK-induced renal damage.** Mice were pretreated with the NF-κB2 inhibitor SN52 0.7 mg/ day (two doses; day -1; day 0) starting 48 hours before administration of TWEAK 0.5 µg, and were sacrificed 24 hours after TWEAK administration. **A.** p52 immunohistochemistry in paraffin-embedded kidney sections. Representative animal from each group (magnification 200x). **B** and **C.** Western blot for p52 **(B)** and IκBα phosphorylation **(C).** Data expressed as mean ± SEM of 8-10 animals per group. *p<0.05 vs control. # p<0.05 vs TWEAK.

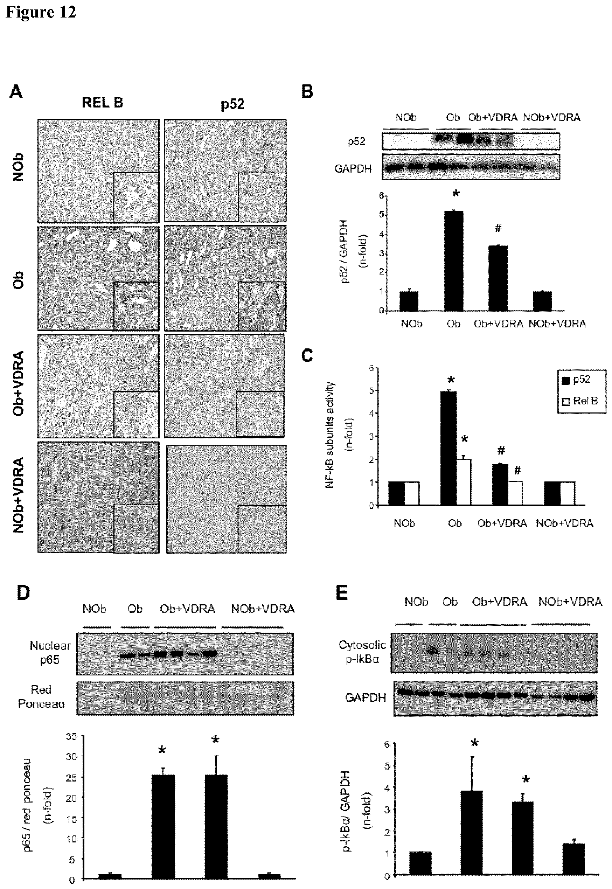

**Figure 12****. Paricalcitol inhibits NF-xB2, but not NF-κB1 activation in experimental Unilateral Ureteral Obstruction (UUO) in mice.** Mice were treated with paricalcitol 750 ng/Kg/day, starting 24 hours before UUO, and studied 5 days after UUO. **A.** Immunohistochemistry in paraffin-embedded kidney sections. Representative animal from each group (magnification 200x). **B.** Western blot, NF-κB2 activation was assessed as p52 levels in total protein extracts. **C.** In isolated renal nuclear proteins, p52 and RelB DNA binding activities were measured by ELISA. **D** and **E.** Western blot assessment of nuclear levels of p65 **(D)** and cytosolic IκBα phosphorylation **(E)** were used to evaluate NF-κB1 pathway activation. In each mouse, obstructed (Ob) kidneys were compared to the corresponding contralateral non-obstructed (Nob) kidney. Data expressed as mean±SEM of 6-8 animals per group. *p<0.05 vs contralateral #p<0.05 vs obstructed kidneys.

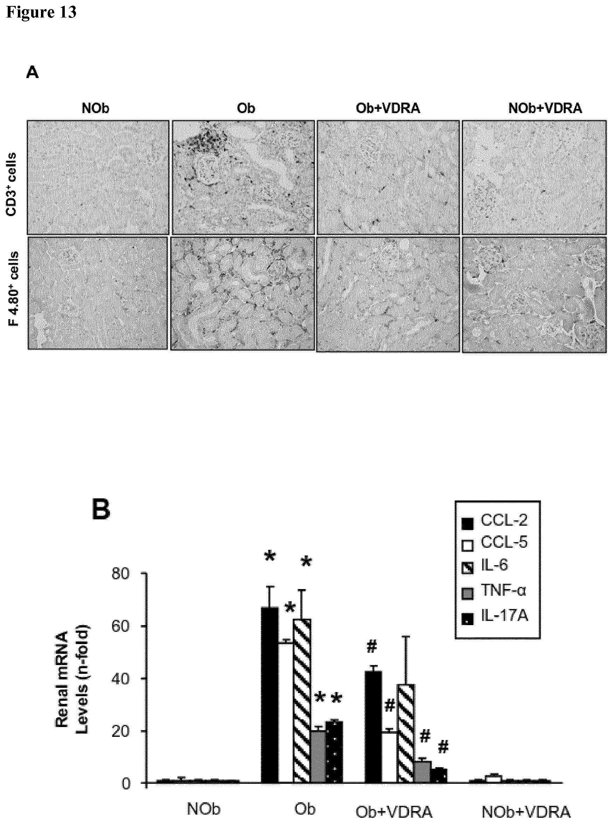

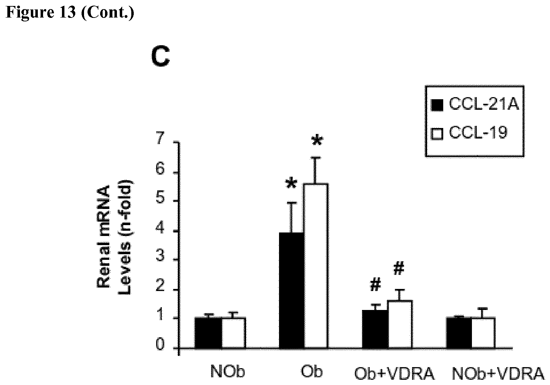

**Figure 13****. Paricalcitol decreases renal inflammation in experimental Unilateral Ureteral Obstruction (UUO) in mice.** Mice were treated with paricalcitol 750 ng/Kg/day, starting 24 hours before UUO, and studied 5 days after UUO. In paraffin-embedded kidney sections, immunohistochemistry using anti-F4/80 and anti-CD3 identified monocyte/macrophages and T lymphocytes, respectively. **A.** Representative animal from each group. Magnification 200X. **B, C.** In RNA obtained from total renal extracts, proinflammatory gene expression (CCL2, CCL5, IL-6, TNF-α and IL-17A) and specific NFκB2-regulated gene expression (CCL19 and CCL21) were determined by Real Time PCR. Data expressed as mean±SEM of 6-8 animals per group. *p<0.05 vs contralateral non-obstructed (NOb) kidney; #p<0.05 vs obstructed (Ob) kidneys

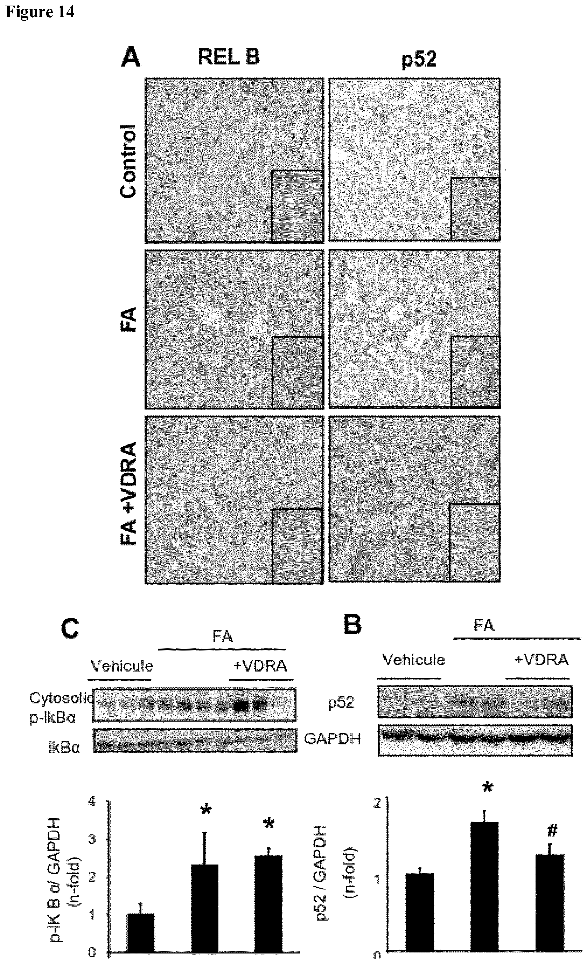

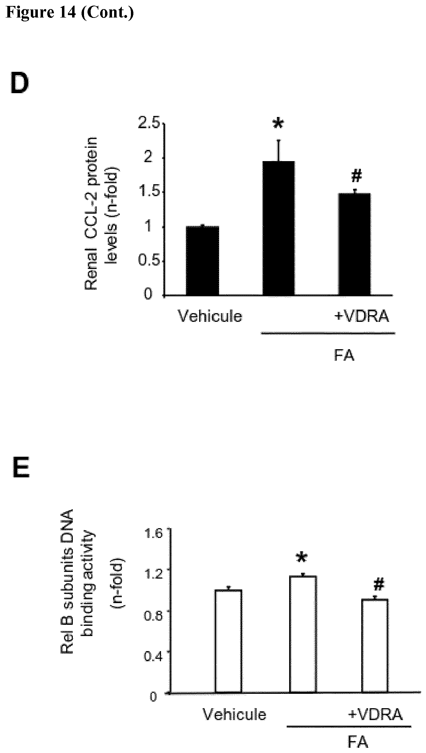

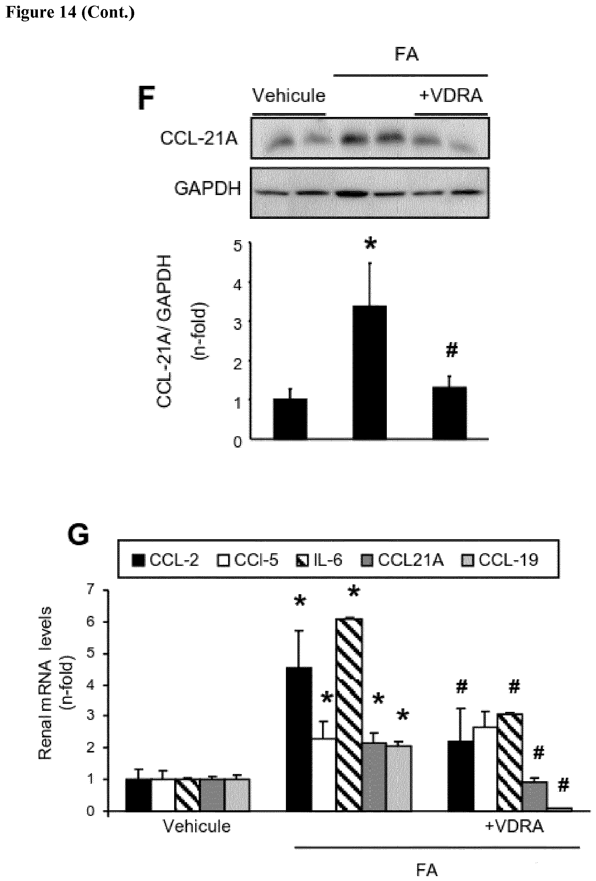

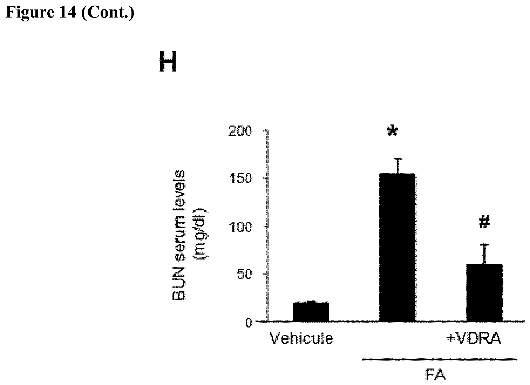

**Figure 14****. Paricalcitol inhibits NF-xB2, but not NF-κB1 activation in folic acid-induced renal injury.** Mice were treated with paricalcitol 25 µg/Kg/day starting 24 hours before folic acid (FA) 300 mg/kg or vehicle (sodium bicarbonate 0.3 mol/L) adminsitration, and studied 24 hours after FA injection. **A.** Immunohistochemistry disclosed nuclear localization of p52 and RelB that was decreased by paricalcitol. Representative animal from each group (magnification 200x). **B and C.** Western blotting of p52, as evidence of NF-κB2 activation **(B)** and IκBα phosphorylation as evidence of NF-κB1 activation **(C). D.** CCL2 protein levels evaluated by ELISA. **E.** In isolated renal nuclear proteins, RelB DNA binding activity was assessed by ELISA. **F.** CCL21 protein levels evaluated in total renal protein extracts by Western Blot. **G.** RNA was obtained from total renal extracts, and proinflammatory gene expression levels were determined by Real Time PCR. **H.** Data of serum BUN levels are shown. Data expressed as mean±SEM of 6-8 animals per group. *p<0.05 vs control; #p<0.05 vs folic acid kidneys.

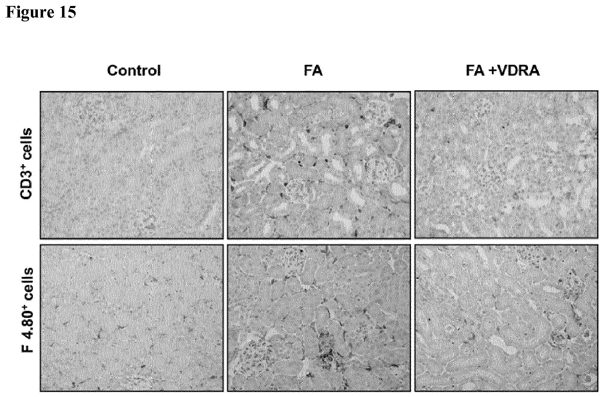

**Figure 15****. Paricalcitol decreases renal inflammation induced by folic acid in mice.** Mice were treated with paricalcitol 25 µg/Kg/day, starting 24 hours before folic acid 300 mg/kg or vehicle (sodium bicarbonate 0.3 mol/L), and studied 24 hours after folic acid administration. Immunohistochemistry in paraffin-embedded kidney sections using anti-F4/80 and anti-CD3 antibodies that identify monocyte/macrophages and T lymphocytes, respectively. Figure shows a representative animal from each group. Magnification 200X.

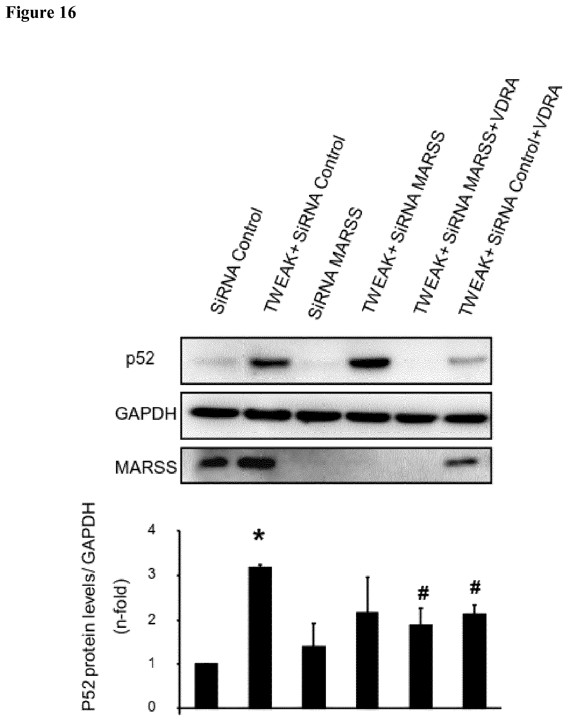

**Figure 16****. Paricalcitol inhibits TWEAK-induced upregulation of specific NF-xB2 targets in MARSS gene silenced cells (other possible receptor of vitamin D).** MARSS gene silencing was achieved in cultured cells using a predesigned and validated SiRNA againts MARSS. Cells were stimulated with recombinant human soluble TWEAK 100 ng/ml. In some experiments cells were preincubated for 48 hours with paricalcitol 15 µmol/L prior to TWEAK stimulation. A. NF-κB2 pathway activation was assessed by western blot of NFκB2 p52. Data expressed as mean±SEM of 3-5 independent experiments. *p<0.05 vs control; #p<0.05 vs TWEAK-treated cells.

### Detailed description of the invention

### Example 1. Material and methods.

### Example 1.1 Experimental models.

All animal procedures were performed according to the guidelines of animal research in the European Community and with prior approval by the Ethics Committee of the Health Research of the IIS-Fundación Jiménez Diaz. For *in vivo* studies, mice received a daily intraperitoneal injection of the VDRA Paricalcitol starting 48 hours prior to induction of kidney damage.

At the time of sacrifice, animals were anesthetized with 5 mg/kg xylazine (Rompun, Bayer AG) and 35 mg/kg ketamine (Ketolar, Pfizer) and the kidneys perfused *in situ* with cold saline before removal. Then, kidney portions were fixed in formalin buffer for immunohistochemistry studies or immediately frozen in liquid nitrogen for gene and protein studies.

TWEAK administration: C57BL/6 female mice (9-12 weeks, weight 20 g, 7-8 animals per group), received a single intraperitoneal injection of 0.5 µg TWEAK dissolved in saline and were sacrificed 24 hours later, as previously described (Sanz et al. 2008). Controls were injected with the saline vehicle (n=8-10 mice per group). TWEAK endotoxin level were <0.1 ng/mg, confirmed by MALDI-TOF. Some animals received paricalcitol 750 ng/kg/day, or the NF-κB2 inhibitor SN52, 0.7 mg/mouse/day, starting 48 hours before TWEAK administration. Animals were sacrificed 24 hours later. The dose of paricalcitol was chosen based on prior experience in mild inflammatory kidney conditions.

Unilateral ureteral obstruction (UUO) was established in male C57BL/6 mice, under isoflurane-induced anesthesia. The left ureter was ligated with silk (5/0) at two locations and cut between ligatures to prevent urinary tract infection (obstructed kidney).(Ucero et al 2014; Rodrigues-Díez et al 2013) Two groups were studied; untreated or paricalcitol treated (750 ng/kg/day, i.p., Abbot), n=6-8 mice per group, starting 48 hours before surgery. Studies compared both kidneys (contralateral vs. obstructed) in each mouse.

Folic acid nephropathy is a classical model of kidney tubulointerstitial injury and inflammation that has been reported in humans. Mice received a single intraperitoneal injection of folic acid (Sigma) 300 mg/kg in 0.3 mol/l sodium bicarbonate or vehicle, and were sacrificed 48 hours later. Two groups were studied; untreated or paricalcitol treated (25 µg/kg/day, i.p., Abbot), n=7-8 per group starting 48 hours before FA injection. The dose of paricalcitol was chosen based on dose-finding preliminary experiments.

### Example 1.2. Cultured cells.

Human kidney proximal tubule epithelial cells (HK2 cell line, ATCC CRL-2190) were grown in RPMI 1640 with 10% fetal bovine serum (FBS), 1% glutamine, 100 U/ml penicillin, 100 µg/ml streptomycin, 5 µg/ml insulin-transferrin-selenite and 36 ng/ml hydrocortisone in 5% CO₂ at 37° C. When cells reached 60 to 70% confluence, they were serum-depleted for 24 hours before the experiment.

Tubulo-epithelial proximal murine cells (MCT cell line) were originally obtained from Dr. Eric Neilson (Vanderbilt University), and used for gene expression studies. These cells were grown in RPMI 1640 with 10% FBS, 1% glutamine, 100 U/ml penicillin, 100 µg/ml streptomycin in 5% CO₂ at 37°C. When cells reached 60 to 70% confluence, they were maintained in RPMI with 1% FBS for 24 hours.

Cells were stimulated with 100ng/ml recombinant human soluble TWEAK (Millipore) or p-200 µg/ml cresyl-sulfate (Raymond Vanholder and Griet Glorieux, University of Gent, Belgium). Concentrations of TWEAK and p-cresyl-sulfate were based on previously published dose-response experiments (Poveda et al. 2014; Sanz et al. 2008). In some experiments cells were preincubated for 48 hours with the paricalcitol 12 µmol/L (Abbott) prior to stimulation. DMSO, used as a solvent in some cases, had no effect on cell viability or on gene expression levels.

### Example 1.3. PBMCs isolation.

Intracellular protein and mRNA levels were measured in PBMCs from ESRD patients on hemodialysis. Inclusion criteria were as follows: age >45 years; male sex; informed consent; and absence of active inflammatory, infectious, or malignant diseases at the initiation or during the study. This study was approved by the IIS-Fundación Jiménez Diaz Ethics Committee. Patients were enrolled after providing written informed consent. PBMCs were separated from whole blood samples (30 ml into EDTA tubes) by a density gradient centrifugation method using Ficoll density gradient centrifugation. In total, 50×10⁶ of PBMCs were obtained from 30 ml of whole blood and 6×10⁶ PBMCs were used to isolate protein, RNA and nuclear/cytosolic protein levels using the NE-PER Reagent (Pierce) following the manufacturer's instructions.

### Example 1.4. Renal histology and immunohistochemistry.

Paraffin-embedded kidney sections (3 µm) were stained using conventional methods. Antigen retrieval was performed by PTlink link system (Dako Diagnostics) with sodium citrate buffer (10 mmol/L) adjusted to pH 6-9 depending on the immunohistochemical marker, followed by immunohistochemical staining in a Dako Autostainer. Steps: 1) endogenous peroxidase blockade; 2) primary antibodies incubation; anti-CD3 (1:300; Dako) or anti-F4/80 (1:5000; Serotec); NF-κB p-p65 (1:200; Cell Signalling); NF-κB2 p52 (1:50; Cell Signaling); RelB (1:50; Santa Cruz Biotechnology); CCL21 (1:90; Santa Cruz Biotechnology); 3) washing; 4) DUOFLEX Doublestain EnVision^{™} treatment, using 3,3'-diaminobenzidine as chromogen. For F4/80 staining a rabbit anti-rat linker was used before EnVision. Sections were counterstained with Carazzi's hematoxylin. The intensity of the reactive mark was obtained using Image-Pro Plus software. For each sample (processed by duplicate in a blinded manner), the average value was obtained from the analysis of 4 fields (20X objective) as density/mm2 or percentage stained area vs total analyzed area. Data are expressed as fold increase over control mice, as mean ± SEM of 8-10 animals per group. Negative controls include non-specific immunoglobulin and no primary antibody.

### Example 1.5. Protein studies.

Proteins were obtained from treated cells or mouse kidneys using lysis buffer (50 mmol/L Tris-HCl, 150 mol/L NaCl, 2mmol/L EDTA, 2 mmol/L EGTA, 0.2% Triton X-100, 0.3% IGEPAL, 10 µl/ml proteinase inhibitors cocktail, 0.2 mmol/L PMSF, and 0.2 mmol/L orthovanadate). To determine protein content the BCA method was used.

For western blot, cell (25 µg/lane) and kidney (100-150 µg/lane) protein extracts were separated on 6%-12% polyacrylamide-SDS gels under reducing conditions. Samples were then transferred onto nitrocellulose membranes (BioRad), blocked with TBS/5% defatted milk/0.05% Tween-20, and incubated overnight at 4°C with the following antibodies (dilution): anti-NF-κB2 p100/p52 (1:500), NF-κB p-p65 (1:500) and NF-κB p65 (1:500, Cell Signalling); CCL-21A (sc-25445, 1:500); RelB (sc-226, 1:500); p-IKKα (sc-101706, 1:500); p-IκBα (sc-8404, 1:500), IκBα (sc-371, 1:500); TRAF3 (sc-6933, 1:250); CIAP-1 (sc-271419, 1:500); NIK (sc- 7211, 1:500); VDR (sc-1008, 1:250, Santa Cruz Biotechnology). Membranes were subsequently incubated with peroxidase-conjugated IgG secondary antibody and developed using an ECL chemiluminiscence kit (Amersham). Loading controls included anti-GAPDH (1:10000; Chemicon), anti-α tubulin (1:5000; Sigma-Aldrich), or anti-histone H1 (sc-8030, 1:250, Santa Cruz Biotechnology) for nuclear proteins or total protein levels in phosphorylation studies. Autoradiographs were scanned using the Gel Doc^{™} EZ imager and analyzed with the Image Lab 3.0 software (BioRad).

ELISA was used to evaluate levels of the chemokines CCL2 and CCL5 (eBioscience). In renal samples, total protein content was determined by the BCA method, and equal amounts of protein were analyzed. Data are expressed as n-fold increase over the mean of control levels.

### Example 1.6. Gene expression studies.

Total RNA was isolated from cells and mouse kidney samples with Trizol (Invitrogen). The cDNA was synthesized using the High-Capacity cDNA Archive Kit (Applied Biosystems) using 2 µg total RNA primed with random hexamer primers. Multiplex Real-Time PCR was performed using Applied Biosystems expression assays for murine and human samples: CCL2 Mm00441242_m1; CCL5 Mm_ 01302428_m1; IL-6 Mm_00446190_m1; CCL19 Mm_00839967_g1, CCL21A Mm_036466971_gH; TRAF3 Hs_00936781_m1; CCL19 -Hs_00171149_m1; CCL21A Hs_00989654_m1. Data were normalized to 18S; 4210893E (VIC) and GAPDH Mm99999915_g1. The mRNA copy numbers were calculated for each sample by the instrument software using Ct value. Results are expressed in copy numbers, calculated relative to unstimulated cells or control mice, after normalization against 18S.

### Example 1.7. ELISA-based NF-xB2 and RelB assay.

Nuclear and cytoplasmic fractions were separated from renal tissues using the NE-PER Reagent (Pierce) following the manufacturer's instructions. In renal nuclear extracts, NF-κB2 and RelB DNA binding activity was measured by its binding to an oligonucleotide containing the NF-κB consensus site using a TransAM NF-κB Family kit (Active Motif) with an antibody that only recognizes active NF-κB2 p52 and RelB.

### Example 1.8. Activation gene expression by Magnetofection.

Gene overexpression was performed in cultured cells using the activation TRAF3CRISPR/Cas9 DNA plasmid (Santa Cruz Biotechnology). Subconfluent cells were transfected with magnetofection^{™} (OZ biosciences) mixture [PolyMag CRISPR magnetofection reagent (1 µl) + DNA plasmid (1 µg)] for 30 min above the magnetic plate according to the manufacturer's instructions. Then, cells were incubated with 10% heat-inactivated FBS for 24 hours and incubated in serum-free medium for 24 hours before the experiments. Cells were stimulated with recombinant human soluble TWEAK (Millipore). In some experiments cells were preincubated for 48 hours with paricalcitol 12 µmol/L (Abbott) prior to stimulation. NF-κB2 pathway activation was assessed by gene expression and Western blot analysis using 1:200 anti-NF-κB2 p100/p52 and1:200 CCL21A. Anti-TRAF3 (1:250, Santa Cruz Biotechnology) was used for overexpression specificity and efficiency assessment, and anti-GAPDH (1:10000) as loading control.

### Example 1.9. Gene silencing.

Gene silencing in cultured cells was performed using a pre-designed siRNA corresponding to VDR (Stealth Select RNAiTM, Ambion). Subconfluent cells were transfected for 24h with 25 nmol/L siRNA using 50 nmol/L Lipofectamine RNAiMAX (Invitrogen, according to the manufacturer's instructions. Then, cells were incubated with 10% heat-inactivated FBS for 24 h, followed by 24 h in serum-free medium.

### Example 1.10. Coimmunoprecipitation assays.

Cells growing in tissue culture dish plates, were lysed in 300-500 µl Triton-NP-40 lysis buffer [50 mmol/L Tris-HCl pH 8, 150 mmol/L NaCl, 1 mmol/L phenylmethylsulphonylfluoride, 1% NP-40/IGEPAL, and a phosphatase-inhibitor cocktail (Set II, Calbiochem)], scraped off the dish and incubated 1 hour to 4°C with shaking. Cell lysates were precleared by incubating with 10 µl protein A-agarose bead slurries (0.5 mL agarose/2 mL phosphate-buffered saline) for 30 min at 4°C, and then centrifuged three times for 5 minutes at 2500 rpm, to wash supernatants. Precleared lysates were incubated with 2.5-5 µg mouse monoclonal anti-TRAF3 antibody (sc-6933, Santa Cruz Biotechnology) overnight at 4°C. The immune complexes were captured by the addition of protein A/G PLUS-agarose (20 µl) bead slurries for 1 hour at 4°C. The agarose beads were collected by centrifugation, washed three times with lysis buffer, resuspended in 2x Laemmli sample buffer, boiled for 5 min, and subjected to SDS-PAGE. Then Western blot was performed as described above, using 1:250 anti-ubiquitin Lys48 specific (#05-1307, Millipore) or 1:250 anti-TRAF3 (sc-6933, Santa Cruz Biotechnology) antibodies.

### Example 1.11. Statistical Analysis.

All results are expressed as mean ± SEM. Differences between intervention groups and controls were assessed by Mann-Whitney test. P<0.05 was considered significant. Statistical analysis was conducted using the SPSS statistical software (version 11.0).

### Example 2. Results.

### • TRAF3 as a reliable biomarker of renal damage.

### Example 2.1. TRAF3 is downregulated in injured kidneys in different experimental models of renal damage.

First, we evaluated changes in TRAF3 protein levels in different experimental models of renal damage. In different experimental models of renal damage in mice, including folic acid-induced renal injury, TWEAK-mediated renal inflammation and unilateral ureteral obstruction, TRAF3 levels were downregulated in injured kidneys, as determined by western blot in total renal proteins **(****Figure 1A; 1B** and **1C****).** Next, we evaluated the direct effect of mediators of renal damage in TRAF3 modulation. Studies were done in cultured human tubular epithelial cells, as representative renal cells that express components of the non-canonical NF-κB2 pathway that promote kidney injury and in PBMCs from healthy donors. Cells were stimulated with tumor necrosis factor-like weak inducer of apoptosis (TWEAK), one of the few cytokines that can activate both NF-κB pathways. TWEAK stimulation downregulated TRAF3 levels, both in renal and PBMCs cells **(****Figure 1D** and **1E****).**

### Example 2.2. TRAF3 modulates the noncanonical NF-xB2 pathway and proinflammatory cytokines.

To investigate the functional consequences of TRAF3 modulation, cells were transfected with a CRISPR/Cas9 TRAF3 activation plasmid **(****Figure 2A****).** TRAF3 overexpression in HK2 cells blocked TWEAK-induced p52 activation **(****Figure 2B****),** as well as the increased expression of proinflammatory genes such as CCL2, CCL5 and IL6 **(****Figure 2C****)** as well as NF-κB2-regulated genes **(****Figure 2D****).**

### Example 2.3. TRAF3 is downregulated in circulating cells from patients with chronic kidney disease.

Next, we investigated TRAF3 levels in ESRD patients, using PBMC the major cell type involved in the pathogenesis of inflammatory diseases. In PBMCs from ESRD patients, TRAF3 protein was downregulated compared to healthy controls **(****Figure 3A****),** whereas TRAF3 mRNA levels were upregulated, suggesting that TRAF3 protein levels modulation occurs by post-traductional modifications **(****Figure 3B****).**

In summary, our preclinical data shows that in response to renal damage, TRAF3 levels are downregulated in cytokine-stimulated renal and PBMCs cells as well as in the injured murine kidneys. Moreover, in circulating cells from ESRD patients TRAF3 levels were also downregulated. All these data shows that TRAF3 is a reliable biomarker of renal damage.

### Example 2.4. TRAF3 downregulation in circulating cells from patients with chronic kidney disease is related to progression to ESRD.

On the other hand, we investigated TRAF3 levels in two groups of CKD patients, one group of patients that not progress to ESRD and other group that progress. We used PBMCs from CKD patients belong to those groups. TRAF3 protein was clearly downregulated in samples from progressing patients compared to non-progressing patients.

In summary, our preclinical data shows that TRAF3 levels are downregulated in circulating cells from ESRD progressing patients, compare to CKD patients that not progress to ESRD. All these data shows that TRAF3 is a reliable biomarker of renal damage progression.

### • TRAF3 levels are modulated by therapeutic treatments with vitamin D analogues or derivatives.

### Example 2.5. Evaluation of TRAF3 modulation by VDRAs in ESRD patients.

In PBMCs of paricalcitol-treated ESRD patients on hemodialysis, TRAF3 protein levels were restored to values in the range of healthy controls, whereas no changes were found in TRAF3 mRNA levels, suggesting that paricalcitol prevents TRAF3 degradation **(****Figure 3A**, 3B).

### Example 2.6. Evaluation of TRAF3 modulation by VDRAs in cytokine-stimulated cells and in injured kidneys

In cultured tubular epithelial cells (HK2 cell line), preincubation with paricalcitol restored TWEAK-induced TRAF3 protein levels to values similar to control-untreated cells **(****Figure 1D****).** Accordingly, in PBMCs from healthy donors, paricalcitol inhibited TWEAK-induced TRAF3 degradation **(****Figure 1E****).**

In different experimental models of renal damage in mice (folic acid-induced renal injury, TWEAK-mediated renal inflammation and unilateral ureteral obstruction), daily treatment with paricalcitol, starting one day before kidney damage, restored renal TRAF3 protein levels to values of control mice **(****Figure 1A; 1B** and **1C****).**

Next, we investigated upstream mechanisms involved in TRAF3 degradation. In PBMCs from ESRD patients, TRAF3 immunoprecipitation studies showed increased TRAF3 ubiquitination in the K48-linked chains, leading to the formation of CIAP1-TRAF3 complexes **(****Figure 4A****).** In paricalcitol-treated patients, TRAF3 ubiquitination and CIAP1-TRAF3 interaction were significantly lowering to values in the range of healthy controls **(****Figure 4A****).** Moreover, CIAP-1 levels were higher in whole protein extracts of PBMCs from ESRD patients than from healthy controls, while levels in paricalcitol-treated ESRD patients were close to control values **(****Figure 4A****).** Similar results were observed in cytokine-stimulated cultured cells. In HK2 cells, TWEAK stimulation induced TRAF3 ubiquitination and CIAP1-TRAF3 complex formation and this was prevented by paricalcitol **(****Figure 4B****).**

### Example 2.7. TRAF3 modulation by paricalcitol was linked to non-canonical NF-κB2 pathway activation and inflammation.

- The VDRA paricalcitol inhibits the non-canonical NF-κB2 pathway activation without modulating the canonical NF-κB1 pathway in PBMCs from CKD patients.

In PBMCs from ESRD patients on hemodialysis, both the canonical and non-canonical NF-κB pathways were activated, as demonstrated by increased p65/NF-κB1 and RelB or p52/NF-κB2 DNA binding activities **(****Figure 5A** and **5B****).** Interestingly, in PBMCs from paricalcitol-treated ESRD patients p65/NF-κB1 activation was similar to non-paricalcitol ESRD treated patients, whereas the NF-κB2 pathway activation was inhibited **(****Figure 5A** and **5B****).** These data indicates that paricalcitol only inhibits the non-canonical NF-κB2 activation.
- Paricalcitol decreases cytokine-induced proinflammatory cytokines in ESRD patients.

In PBMCs from ESRD hemodialysis patients, proinflammatory gene expression levels were increased, but were significantly lower in ESRD patients treated with paricalcitol **(****Figure 5C****).** This observation supports the anti-inflammatory properties of paricalcitol in human CKD.

### Example 2.8. Evaluation of TRAF3 modulation by VDRAs in cells and its potential relation to the regulation of NF-κB/inflammatory pathways.

- Paricalcitol only regulates NF-κB2 pathway activation in cytokine-stimulated cultured cells

In cultured tubular epithelial cells (HK2 cell line), TWEAK increased p65 phosphorylation and IκBα phosphorylation and downregulated cytosolic IκBα. levels **(****Figure 6A** and **6B****).** Preincubation with paricalcitol did not modulate TWEAK-induced NF-κB1 activation **(****Figure 6A** and **6B****),** while paricalcitol inhibited TWEAK-induced IKK-α phosphorylation, the increase in NIK, and p52 and RelB nuclear levels **(****Figure 6A****,** **6C** and **6D****).** Thus, paricalcitol inhibited TWEAK-induced activation of the non-canonical NF-κB pathway but did not modulate canonical NF-κB activation.

In PBMCs from healthy donors, paricalcitol inhibited TWEAK-induced NF-κB2 activation without modulating the canonical NF-κB1 pathway **(****Figure 6E****).**

The direct paricalcitol regulation of proinflammatory genes was confirmed in cultured cells. In PBMCs obtained from healthy donors, paricalcitol inhibited TWEAK-induced upregulation of several proinflammatory genes, such as CCL2, CCL5 and IL6, as well as the NF-κB2-controlled genes CCL21A and CCL19 **(****Figure 7A****).** Similar results were obtained in cultured tubular cells **(****Figure 7B** and **7C****).** Moreover, in tubular cells NF-κB2 inhibition with SN52 peptide prevented the TWEAK-induced upregulation of proinflammatory genes **(****Figure 7B** and **7C****),** mimicking paricalcitol effects.

**Example 2.9. Evaluation of TRAF3 modulation by VDRAs in preclinical experimental models of renal damage and its potential relation to the regulation of NF-κB/inflammatory pathways.**
- Paricalcitol decreases TWEAK-induced renal inflammation in mice by modulating non-canonical NF-κB2 pathway activation.

TWEAK administration causes an acute inflammatory response in murine kidneys, and activates both canonical and non-canonical NF-κB pathways. Therefore, we used a model of TWEAK-induced renal inflammation to evaluate the effect of paricalcitol on NF-κB activation in the kidney. In TWEAK-injected mice, paricalcitol significantly decreased the TWEAK-induced upregulation of infiltrating monocytes/macrophages (F4/80⁺ cells), T lymphocytes (CD3+ cells) **(****Figure 8A** and **8B****),** and proinflammatory factors, such as CCL2, CCL5 and IL-6 **(****Figure 8C** and **8D****).** In kidneys from TWEAK-injected mice, IκBα phosphorylation and nuclear p65 NF-κB levels were higher than in controls, but were not modified in response to paricalcitol **(****Figure 9A** and **9B****).** However, paricalcitol inhibited TWEAK-induced NF-κB2 activation in the kidney, as demonstrated by reduced nuclear p52 and RelB accumulation in tubular epithelial cells. **(****Figure 9C** and **9D****).** Moreover, paricalcitol inhibited TWEAK-induced p52 and RelB DNA binding activities **(****Figure 9E****).** In this regard, TWEAK activation of the NF-κB2 pathway in tubular cells leads the synthesis of specific chemokines, such as CCL21A and CCL19. In TWEAK-injected mice paricalcitol reduced renal CCL21A and CCL19 gene expression **(****Figure 10A****)** and CCL21 protein levels **(****Figure 10B****).** Interestingly, CCL21 was located in renal tubules in TWEAK-injected mice **(****Figure 10C** and 10 D), that is, in the same renal structures where active NF-κB2 components RelB and p52 were located **(****Figure 9C****).** These data suggest that paricalcitol anti-inflammatory effects could be mediated by the modulation of NF-κB2 target genes.

In TWEAK-injected mice, the inhibition of the NF-κB2 pathway with SN52, a synthetic peptide that specifically blocks the nuclear translocation of the p52/NF-κB2 subunit **(****Figure 11A** and **11B****),** significantly decreased the number of infiltrating monocytes/macrophages (F4/80⁺cells) and T lymphocytes (CD3⁺) **(****Figure 8A** and **8B****),** thus displaying a clear-cut anti-inflammatory effect. Interestingly, in TWEAK-injected mice, SN52 prevented the upregulation of several proinflammatory genes, including the specific NF-κB2 target genes CCL21A and CCL19 **(****Figure 8E****),** and known to be regulated also by NF-κB1 (IL-6, CCL2, and CCL5) **(****Figure 8C****).** These data clearly demonstrate that NF-κB2 inhibition could be a potential therapeutic option for renal inflammatory diseases.
- Paricalcitol inhibits the non-canonical NF-κB2 pathway in experimental unilateral ureteral obstruction.

In unilateral ureteral obstruction (UUO model), paricalcitol decreases kidney inflammatory cell infiltration and expression of CCL5 and CCL2, without blocking nuclear p65/NF-κB1 translocation. Therefore, we investigated whether paricalcitol modulated the NF-xB2 pathway in UUO. Paricalcitol prevented the increased RelB levels and its nuclear localization observed in obstructed kidneys **(****Figure 12A****).** Moreover, it blocked p100/p52 processing, and p52 nuclear translocation and DNA binding activity **(****Figure 12A**, **12B**·). By contrast, NF-κB1 activation, evaluated by IκB-α phosphorylation and p65/NF-κB DNA binding activity, was not modified in paricalcitol-treated obstructed kidneys **(****Figure 12C****).** Importantly, in obstructed kidneys paricalcitol prevented inflammatory cell infiltration and the increased expression of proinflammatory factors, such as CCL2, CCL5, IL6 **(****Figure 13B****),** and specific NF-κB2-regulated chemokines, including CCL21A and CCL19 **(****Figure 13C****).**
- Paricalcitol inhibits the NF-κB2, but not the NF-κB1 pathway, in experimental folic acid-induced acute kidney injury.

Next, we explored the effect of paricalcitol and the relative contribution of NF-κB2 pathway to folic acid (FA)-induced renal damage in mice. Paricalcitol only blocked NF-κB2 activation, as determined by RelB and p52 levels, nuclear translocation and RelB DNA-binding activity **(****Figure 14A, 14B****,** **14E****)** and the gene and protein expression of NF-κB2-dependent cytokines, such as CCL21A, as well as other pro-inflammatory factors **(****Figure 14F** and **14G****),** whereas NF-κB1 activation, evaluated by IκB-α phosphorylation, was not modified **(****Figure 14C****).** Importantly, paricalcitol also prevented renal inflammatory cell infiltration **(****Figure 15****).** These data suggest that paricalcitol protects from folic acid-induced kidney injury by inhibiting inflammation and the non-canonical NF-κB2 pathway. Additionally, Paricalcitol also restored changes in renal function assessed by serum BUN levels **(****Figure 14H****),** showing the beneficial effect of this VDRA in the acute phase of renal damage in this experimental model of kidney injury.

### Example 2.10. Paricalcitol inhibits NF-xB2 pathway independently of MARSS

In order to test the hypothesis that MARSS could be a receptor for VDRAs, MARSS was blocked by gene silencing approach. HK2 cells were transfected with a MARSS siRNA or a control siRNA and then pre-treated or not with paricalcitol before stimulation with TWEAK. In MARSS silenced-cells paricalcitol diminished TWEAK-induced NF-xB2 activation, suggesting a MARSS-independent effect of paricalcitol **(****Figure 16****).**

## Claims

1. *In vitro* method for detecting renal disease which comprises:
a. Determining at least TRAF3 protein level in peripheral blood mononuclear cells (PBMCs) isolated from blood samples obtained from the patient,
b. Wherein if the level determined in step (a) is statistically lower than the level determined in healthy control subjects, this is an indication that the patient is suffering from renal disease.

2. *In vitro* method, according to claim 1, wherein the renal disease is clinically manifested as acute kidney injury (AKI) or chronic kidney disease (CKD), or the renal disease has progressed to end stage renal disease (ESRD).

3. *In vitro* method for deciding or recommending whether to treat patients suffering from renal disease with vitamin D analogues or derivatives which comprises:
a. Determining at least TRAF3 protein level in peripheral blood mononuclear cells (PBMCs) isolated from blood samples obtained from the patient,
b. Wherein if the level determined in step (a) is statistically lower than the level determined in healthy control subjects, a treatment with vitamin D analogues or derivatives is recommended.

4. *In vitro* method for predicting the response of patients suffering from renal disease to a treatment with vitamin D analogues or derivatives which comprises:
a. Determining at least TRAF3 protein level in peripheral blood mononuclear cells (PBMCs) isolated from blood samples obtained from patients before the administration of the vitamin D analogue or derivative,
b. Wherein if the level determined in step (a) is statistically lower than the expression level determined after the administration of the vitamin D analogues or derivatives, this is an indication that the patient responds to the treatment.

5. *In vitro* method, according to any of the claims 3 or 4, wherein the renal disease is clinically manifested as acute kidney injury (AKI) or chronic kidney disease (CKD), or the renal disease has progressed to end stage renal disease (ESRD).

6. *In vitro* method, according to any of the claims 3 to 5, wherein the vitamin D analogue or derivative is selected from the group comprising: paricalcitol, calcitriol, calcidiol, alfacalcidol, tacalcitol, calcifediol, calcipotriol, maxacalcitol, doxercalciferol and/or falecalcitriol.

7. *In vitro* use of the TRAF3 protein level determined in peripheral blood mononuclear cells (PBMCs) isolated from blood samples obtained from the patient for detecting renal disease, for predicting the response of patients suffering from renal disease to a treatment with vitamin D analogues or derivatives, or for deciding or recommending whether to treat patients suffering from renal disease with vitamin D analogues or derivatives.

## Patentansprüche

1. In-vitro-Verfahren zur Detektion einer Nierenerkrankung, welches Folgendes umfasst:
a. das Bestimmen mindestens des TRAF3-Proteinniveaus in peripheren mononukleären Blutzellen (PBMC) isoliert aus Blutproben, welche aus dem Patienten erhalten werden,
b. wobei, wenn das in Schritt (a) bestimmte Niveau statistisch niedriger als das in gesunden Kontrollindividuen bestimmte Niveau ist, dies ein Anzeichen dafür ist, dass der Patient unter einer Nierenerkrankung leidet.

2. In-vitro-Verfahren nach Anspruch 1, wobei sich die Nierenerkrankung klinisch als akute Nierenschädigung (AKI) oder chronische Nierenerkrankung (CKD) äußert, oder die Nierenerkrankung zu Nierenerkrankung im Endstadium (ESRD) fortgeschritten ist.

3. In-vitro-Verfahren zum Entscheiden oder Empfehlen, ob die Patienten, welche unter einer Nierenerkrankung leiden, mit Vitamin D-Analogen oder -Derivaten behandelt werden sollen, welches Folgendes umfasst:
a. das Bestimmen mindestens des TRAF3-Proteinniveaus in peripheren mononukleären Blutzellen (PBMC) isoliert aus Blutproben, welche aus dem Patienten erhalten werden,
b. wobei, wenn das in Schritt (a) bestimmte Niveau statistisch niedriger als das in gesunden Kontrollindividuen bestimmte Niveau ist, eine Behandlung mit Vitamin D-Analogen oder -Derivaten empfohlen wird.

4. In-vitro-Verfahren zum Vorhersagen der Antwort der Patienten, welche unter einer Nierenerkrankung leiden, auf eine Behandlung mit Vitamin D-Analogen oder - Derivaten, welches Folgendes umfasst:
a. das Bestimmen mindestens des TRAF3-Proteinniveaus in peripheren mononukleären Blutzellen (PBMC) isoliert aus Blutproben, welche aus Patienten erhalten werden, vor der Verabreichung des Vitamin D-Analogs oder -Derivats,
b. wobei, wenn das in Schritt (a) bestimmte Niveau statistisch niedriger als das Expressionsniveau ist, welches nach der Verabreichung der Vitamin D-Analoge oder -Derivate bestimmt wird, dies ein Anzeichen dafür ist, dass der Patient auf die Behandlung anspricht.

5. In-vitro-Verfahren nach einem der Ansprüche 3 oder 4, wobei die Nierenerkrankung sich klinisch als akute Nierenschädigung (AKI) oder chronische Nierenerkrankung (CKD) äußert, oder die Nierenerkrankung zu Nierenerkrankung im Endstadium (ESRD) fortgeschritten ist.

6. In-vitro-Verfahren nach einem der Ansprüche 3 bis 5, wobei das Vitamin D-Analog oder -Derivat aus der Gruppe umfassend Paricalcitol, Calcitriol, Calcidiol, Alfacalcidol, Tacalcitol, Calcifediol, Calcipotriol, Maxacalcitol, Doxercalciferol und/oder Falecalcitriol ausgewählt wird.

7. In-vitro-Verwendung des TRAF3-Proteinniveaus, welcher in peripheren mononukleären Blutzellen (PBMC) bestimmt wird, isoliert aus Blutproben, welche aus dem Patienten erhalten werden, zur Detektion einer Nierenerkrankung, zum Vorhersagen der Antwort der Patienten, welche unter einer Nierenerkrankung leiden, auf eine Behandlung mit Vitamin D-Analogen oder -Derivaten, oder zum Entscheiden oder Empfehlen, ob die Patienten, welche unter einer Nierenerkrankung leiden, mit Vitamin D-Analogen oder -Derivaten behandelt werden sollen.

## Revendications

1. Procédé *in vitro* pour la détection d'une maladie rénale comprenant :
a. Déterminer au moins le niveau de protéine TRAF3 dans des cellules mononucléaires du sang périphérique (CMSP) isolées depuis des échantillons de sang obtenus du patient,
b. Dans lequel si le niveau déterminé à l'étape (a) est statistiquement plus bas que le niveau déterminé chez des sujets témoins sains, ceci est une indication que le patient souffre de maladie rénale.

2. Procédé *in vitro,* selon la revendication 1, dans lequel la maladie rénale se manifeste cliniquement comme insuffisance rénale aiguë (IRA) ou maladie rénale chronique (MRC), ou la maladie rénale a évolué à maladie rénale en stade terminal (MRST).

3. Procédé *in vitro* pour décider ou recommander si traiter des patients souffrant de maladie rénale avec des analogues de vitamine D ou dérivés, qui comprend :
a. Déterminer au moins le niveau de protéine TRAF3 dans des cellules mononucléaires du sang périphérique (CMSP) isolées depuis des échantillons de sang obtenus du patient,
b. Dans lequel si le niveau déterminé à l'étape (a) est statistiquement plus bas que le niveau déterminé chez des sujets témoins sains, un traitement avec des analogues de vitamine D ou dérivés est recommandé.

4. Procédé *in vitro* pour prédire la réponse de patients souffrant de maladie rénale à un traitement avec des analogues de la vitamine D ou dérivés, qui comprend :
a. Déterminer au moins le niveau de protéine TRAF3 dans des cellules mononucléaires du sang périphérique (CMSP) isolées depuis des échantillons de sang obtenus de patients avant l'administration d'analogues de vitamine D ou dérivés,
b. Dans lequel si le niveau déterminé à l'étape (a) est statistiquement plus bas que le niveau d'expression déterminé après l'administration d'analogues de vitamine D ou dérivés, ceci est une indication que le patient répond au traitement.

5. Procédé *in vitro,* selon l'une quelconque des revendications 3 ou 4, dans lequel la maladie rénale se manifeste cliniquement comme insuffisance rénale aiguë (IRA) ou maladie rénale chronique (MRC), ou la maladie rénale a évolué à maladie rénale en stade terminal (MRST).

6. Procédé *in vitro* selon l'une quelconque des revendications 3 à 5, dans lequel l'analogue de vitamine D ou dérivé est sélectionné depuis le groupe comprenant : paricalcitol, calcitriol, calcidiol, alfacalcidol, tacalcitol, calcifédiol, calcipotriol, maxacalcitol, doxercalciférol et/ou falécalcitriol.

7. Utilisation *in vitro* du niveau de protéine TRAF3 déterminé dans des cellules mononucléaires du sang périphérique (CMSP) isolées depuis des échantillons de sang obtenus du patient pour détecter une maladie rénale, pour prédire la réponse de patients souffrant de maladie rénale à un traitement avec des analogues de vitamine D ou dérivés, ou pour décider ou recommander si traiter des patients souffrant de maladie rénale avec des analogues de vitamine D ou dérivés.
